(19) Europäisches Patentamt
European Patent Office
Office européen des brevets

(11) EP 2 313 343 B1

(12) # FASCICULE DE BREVET EUROPEEN

(45) Date de publication et mention de la délivrance du brevet:
**14.11.2012 Bulletin 2012/46**

(21) Numéro de dépôt: **09784285.0**

(22) Date de dépôt: **21.07.2009**

(51) Int Cl.:
***C01B 39/48*** *(2006.01)*
***B01J 29/76*** *(2006.01)*
***C01B 39/46*** *(2006.01)*
***C10G 45/64*** *(2006.01)*
***B01J 29/78*** *(2006.01)*
***C07C 6/12*** *(2006.01)*
***C10G 45/62*** *(2006.01)*
***B01J 29/74*** *(2006.01)*
***B01J 29/70*** *(2006.01)*
***C07C 1/24*** *(2006.01)*
***C07C 5/27*** *(2006.01)*
***C07C 2/12*** *(2006.01)*
***B01J 37/00*** *(2006.01)*
***C07C 11/02*** *(2006.01)*
***B01J 29/72*** *(2006.01)*

(86) Numéro de dépôt international:
**PCT/FR2009/000899**

(87) Numéro de publication internationale:
**WO 2010/015733 (11.02.2010 Gazette 2010/06)**

(54) **CATALYSEUR COMPRENANT UNE ZEOLITHE IZM-2 ET AU MOINS UN METAL ET SON UTILISATION EN TRANSFORMATION D'HYDROCARBURES**

KATALYSATOR MIT EINEM IZM-2-ZEOLITH UND MINDESTENS EINEM METALL SOWIE SEINE VERWENDUNG ZUR UMWANDLUNG VON KOHLENWASSERSTOFFEN

CATALYST COMPRISING AN IZM-2 ZEOLITE AND AT LEAST ONE METAL AND USE THEREOF IN THE CONVERSION OF HYDROCARBONS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **08.08.2008 FR 0804559**

(43) Date de publication de la demande:
**27.04.2011 Bulletin 2011/17**

(73) Titulaire: **IFP Energies nouvelles**
**92852 Rueil-Malmaison Cedex (FR)**

(72) Inventeurs:
- **GUILLON, Emmanuelle**
  **F-69390 Vernaison (FR)**
- **CADRAN, Nicolas**
  **F-69600 Oullins (FR)**
- **MAURY, Sylvie**
  **F-69390 Charly (FR)**
- **CABIAC, Amandine**
  **F-69007 Lyon (FR)**

(56) Documents cités:
**EP-A- 1 702 888**
**EP-A- 1 953 118**
**WO-A-2009/004131**
**WO-A2-2009/144411**
**WO-A2-2009/144412**
**US-A- 4 539 193**
**US-A- 5 207 893**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte au domaine des catalyseurs zéolithiques et à leur utilisation dans différents procédés de transformation de charges hydrocarbonées. Plus précisément, la présente invention se rapporte à un catalyseur comprenant au moins une zéolithe IZM-2, au moins une matrice et au moins un métal choisi parmi les métaux des groupes VIII, VIB et VIIB.

Etat de la technique antérieure

Les matériaux microporeux cristallisés, tel que les zéolithes ou les silicoaluminophosphates, sont des solides très utilisés dans l'industrie pétrolière en tant que catalyseur, support de catalyseur, adsorbant ou agent de séparation. Bien que de nombreuses structures cristallines microporeuses aient été découvertes, l'industrie du raffinage et de la pétrochimie est toujours à la recherche de nouvelles structures zéolitiques qui présentent des propriétés particulières pour des applications comme la purification ou la séparation des gaz, la conversion d'espèces carbonées ou autres. Les propriétés d'un catalyseur zéolithique dépendent fortement de la structure poreuse de la zéolithe, de sa stabilité et de son acidité.

Le document EP1953118 A décrit un catalyseur comprenant une zeolithe IZM-1, ladite zéolithe présentant une composition chimique, exprimée sur une base anhydre, en termes de moles d'oxydes, par la formule générale suivante : $XO_2 : aY_2O_3 : bM_nO$.

Résumé et intérêt de l'invention

La présente invention porte sur un catalyseur comprenant au moins une zéolithe IZM-2, au moins une matrice et au moins un métal choisi parmi les métaux des groupes VIII, VIB et VIIB de la classification périodique des éléments, ladite zéolithe présentant un diagramme de diffraction des rayons X incluant au moins les raies inscrites dans le tableau 1 et présentant une composition chimique, exprimée sur une base anhydre, en termes de moles d'oxydes, par la formule générale suivante : $XO_2 : aY_2O_3 : bM_{2/n}O$ dans laquelle X représente au moins un élément tétravalent, Y représente au moins un élément trivalent et M est au moins un métal alcalin et/ou un métal alcalino-terreux de valence n, a et b représentant respectivement le nombre de moles de $Y_2O_3$ et $M_{2/n}O$ et a est compris entre 0,001 et 0,5 et b est compris entre 0 et 1.

Ledit catalyseur selon l'invention est avantageusement utilisé pour la mise en oeuvre de différents procédés de transformation de charges hydrocarbonées. En particulier, ledit catalyseur conduit à des performances catalytiques intéressantes lorsqu'il est utilisé dans l'isomérisation des composés aromatiques à 8 atomes de carbone, dans la transalkylation des composés alkylaromatiques, dans l'hydroisomérisation de paraffines linéaires légères et dans la transformation d'alcools.

Description détaillée de l'invention

La présente invention a pour objet un catalyseur comprenant au moins une zéolithe IZM-2, au moins une matrice et au moins un métal choisi parmi les métaux des groupes VIII, VIB et VIIB de la classification périodique des éléments, ladite zéolithe présentant un diagramme de diffraction des rayons X incluant au moins les raies inscrites dans le tableau 1 ci-dessous :

Tableau 1 : Valeurs moyennes des $d_{hkl}$ et intensités relatives mesurées sur un diagramme de diffraction de rayons X de la zéolithe IZM-2 calcinée

| 2 thêta (°) | $d_{hkl}$ (Å) | Irel | 2 thêta (°) | $d_{hkl}$ (Å) | Irel |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | f |
| 14,76 | 6,00 | ff | 24,65 | 3,61 | ff |

(suite)

| 2 thêta (°) | $d_{hkl}$ (Å) | Irel | 2 thêta (°) | $d_{hkl}$ (Å) | Irel |
|---|---|---|---|---|---|
| 15,31 | 5,78 | ff | 26,78 | 3,33 | f |
| 15,62 | 5,67 | ff | 29,33 | 3,04 | ff |
| 16,03 | 5,52 | ff | 33,06 | 2,71 | ff |
| 17,60 | 5,03 | ff | 36,82 | 2,44 | ff |
| 18,22 | 4,87 | ff | 44,54 | 2,03 | ff |

où FF = très fort ; F = fort ; m = moyen ; mf = moyen faible ; f = faible ; ff = très faible et présentant une composition chimique, exprimée sur une base anhydre, en termes de moles d'oxydes, par la formule générale suivante : $XO_2$ : $aY_2O_3$ : $bM_{2/n}O$ dans laquelle X représente au moins un élément tétravalent, Y représente au moins un élément trivalent et M est au moins un métal alcalin et/ou un métal alcalino-terreux de valence n, a et b représentant respectivement le nombre de moles de $Y_2O_3$ et $M_{2/n}O$ et a est compris entre 0,001 et 0,5 et b est compris entre 0 et 1.

Le diagramme de diffraction dont les données figurent dans le tableau 1 est obtenu par analyse radiocristallographique au moyen d'un diffractomètre en utilisant la méthode classique des poudres avec le rayonnement $K\alpha_1$ du cuivre ($\lambda$ = 1,5406Å). A partir de la position des pics de diffraction représentée par l'angle $2\theta$, on calcule, par la relation de Bragg, les équidistances réticulaires $d_{hkl}$ caractéristiques de l'échantillon. L'erreur de mesure $\Delta(d_{hkl})$ sur $d_{hkl}$ est calculée grâce à la relation de Bragg en fonction de l'erreur absolue $\Delta(2\theta)$ affectée à la mesure de 29. Une erreur absolue $\Delta(2\theta)$ égale à $\pm$ 0,02° est communément admise. L'intensité relative $I_{rel}$ affectée à chaque valeur de $d_{hkl}$ est mesurée d'après la hauteur du pic de diffraction correspondant. Le diagramme de diffraction des rayons X de la zéolithe IZM-2 présente dans le catalyseur selon l'invention comporte au moins les raies aux valeurs de $d_{hkl}$ données dans le tableau 1. Dans la colonne des $d_{hkl}$, on a indiqué les valeurs moyennes des distances inter-réticulaires en Angströms (Å). Chacune de ces valeurs doit être affectée de l'erreur de mesure $\Delta(d_{hkl})$ comprise entre $\pm$ 0,6Å et $\pm$ 0,01Å.

La zéolithe IZM-2 présente dans le catalyseur selon l'invention présente une composition chimique exprimée sur une base anhydre, en termes de moles d'oxydes, définie par la formule générale suivante : $XO_2$ : $aY_2O_3$ : $bM_{2/n}O$, dans laquelle X représente au moins un élément tétravalent, Y représente au moins un élément trivalent et M est au moins un métal alcalin et/ou un métal alcalino-terreux de valence n. Dans ladite formule donnée ci-dessus, a représente le nombre de moles de $Y_2O_3$ et a est compris entre 0,001 et 0,5, de préférence entre 0,001 et 0,05 et de manière très préférée entre 0,001 et 0,02 et b représente le nombre de moles de $M_{2/n}O$ et est compris entre 0 et 1, de préférence entre 0 et 0,5 et de manière encore plus préférée entre 0,005 et 0,5.

Conformément à l'invention, X est préférentiellement choisi parmi le silicium, le germanium, le titane et le mélange d'au moins deux de ces éléments tétravalents, très préférentiellement X est le silicium et Y est préférentiellement choisi parmi l'aluminium, le bore, le fer, l'indium et le gallium, très préférentiellement Y est l'aluminium. Dans la zéolithe IZM-2 présente dans le catalyseur selon l'invention, X est préférentiellement le silicium et Y est préférentiellement l'aluminium. M est préférentiellement choisi parmi le lithium, le sodium, le potassium, le rubidium, le césium, le calcium, le magnésium, le baryum et le mélange d'au moins deux de ces métaux et très préférentiellement M est le sodium ou le césium.

La zéolithe IZM-2 présente dans le catalyseur selon l'invention et contenant des atomes X et Y tels que définis ci-dessus, de préférence des atomes d'aluminium et des atomes de silicium, présente un rapport atomique X/Y global, de préférence un rapport atomique Si/Al global, compris entre 5 et 100, de préférence compris entre 10 et 50 et de manière très préférée compris entre 10 et 35. La zéolithe présente dans le catalyseur selon l'invention peut également être désaluminée. La zéolithe IZM-2 présente dans le catalyseur selon l'invention se présente très avantageusement sous sa forme protonée (forme hydrogène $H^+$) dans laquelle la proportion en cation autre que $H^+$ est inférieure à 30% du nombre total de cations, de préférence inférieure à 20% et de manière très préférée inférieure à 5% par rapport au nombre total de cations sur la zéolithe. Conformément à l'invention, lorsque la zéolithe IZM-2 se trouve sous sa forme protonée, le coefficient b est nul dans la formule $XO_2$ : $aY_2O_3$ : $bM_{2/n}O$ donnée ci-dessus.

Conformément à l'invention, ledit catalyseur comprend au moins un métal choisi parmi les métaux des groupes VIB, VIIB et VIII de la classification périodique des éléments. Ledit catalyseur comprend soit un métal choisi parmi les métaux des groupes VIB, VIIB et VIII soit plusieurs métaux en mélange choisis parmi les métaux des groupes VIB, VIIB et VIII. Parmi les métaux du groupe VIB, le chrome et le molybdène sont préférés. Parmi les métaux du groupe VIIB, le rhénium est préféré. Parmi les métaux du groupe VIII, le nickel, le ruthénium, le rhodium, le palladium, l'osmium, l'iridium et le platine sont préférés, et de manière très préférée ledit métal du groupe VIII est choisi parmi le palladium, le nickel et le platine.

La matrice présente dans le catalyseur selon l'invention est une matrice minérale poreuse, généralement

amorphe. Elle est choisie parmi les éléments du groupe formé par les alumines, les silices, la magnésie, les silices-alumines amorphes, les argiles naturelles (kaolin, bentonite, sepiolite, attapulgite), l'oxyde de titane, l'oxyde de bore, la zircone, les phosphates d'aluminium, les phosphates de titane, les phosphates de zirconium, le charbon et leurs mélanges. On préfère utiliser une matrice contenant de l'alumine, particulièrement sous toutes ses formes connues de l'Homme du métier, et de manière encore plus préférée l'alumine gamma. On peut aussi avantageusement utiliser des mélanges d'alumine et de silice, des mélanges d'alumine et de silice-alumine.

Ledit catalyseur selon l'invention comprend également avantageusement au moins un métal additionnel choisi parmi les métaux des groupes IIIA et IVA de la classification périodique des éléments et de préférence choisi parmi le gallium, l'indium, l'étain et leur mélange et de manière très préférée choisi parmi l'indium, l'étain et leur mélange. En particulier, le catalyseur selon l'invention comprend avantageusement au moins un métal du groupe VIII de la classification périodique des éléments, préférentiellement du platine ou du palladium, et au moins un métal choisi parmi les métaux des groupes IIIA et IVA, de préférence de l'indium et l'étain.

Le catalyseur selon l'invention est dépourvu de toute phase sulfure.

Ledit catalyseur selon l'invention contient plus particulièrement :

- de 1 à 90%, de préférence de 3 à 80% et de manière encore plus préférée de 4 à 60% poids de ladite zéolithe IZM-2,
- de 0,01 à 4%, de préférence de 0,05 à 2% poids d'au moins un métal choisi parmi les métaux des groupes VIB, VIIB et VIII de la classification périodique des éléments,
- éventuellement de 0,01 à 2%, de préférence de 0,05 à 1% poids d'au moins un métal additionnel choisi parmi les métaux des groupes IIIA et IVA de la classification périodique des éléments,
- au moins une matrice assurant le complément à 100% dans le catalyseur.

Ledit catalyseur selon l'invention se présente sous forme de billes ou d'extrudés, de préférence sous forme d'extrudés. Il présente des propriétés mécaniques telles que la valeur de l'écrasement en lit, déterminé selon la méthode Shell (SMS 1471-74), est de préférence supérieure à 0,7 MPa.

La présente invention a également pour objet la préparation du catalyseur selon l'invention. La préparation du catalyseur selon l'invention commence d'abord par la préparation de la zéolithe IZM-2.

Ladite zéolithe IZM-2 présente dans le catalyseur selon l'invention est préparée selon un procédé dans lequel on fait réagir un mélange aqueux comportant au moins une source d'au moins un oxyde $XO_2$ au moins une source d'au moins un oxyde $Y_2O_3$, au moins une source d'au moins un métal alcalin et/ou alcalino-terreux, au moins une espèce organique R comportant deux atomes d'azote quaternaires, le mélange présentant préférentiellement la composition molaire suivante :

$XO_2/Y_2O_3$ : au moins 2, de préférence au moins 20, de manière plus préférée de 55 à 600,
$H_2O/XO_2$ : 1 à 100, de préférence de 10 à 70,
$R/XO_2$: 0,02 à 2, de préférence de 0,05 à 0,5,
$M_{2/n}O/XO_2$: 0,001 à 1, de préférence de 0,005 et 0,5,
où X est un ou plusieurs élément(s) tétravalent(s) choisi(s) dans le groupe formé par les éléments suivants : silicium, germanium, titane, de préférence le silicium, où Y est un ou plusieurs élément(s) trivalent(s) choisi(s) dans le groupe formé par les éléments suivants : aluminium, fer, bore, indium et gallium, de préférence l'aluminium et où M est un ou plusieurs metal(aux) alcalin(s) et/ou alcalino-terreux choisi(s) parmi le lithium, le sodium, le potassium, le rubidium, le césium, le calcium, le magnésium, le baryum et le mélange d'au moins deux de ces métaux de préférence le sodium.

R est une espèce organique ayant deux atomes d'azote quaternaires jouant le rôle de structurant organique. Préférentiellement, R est le composé azoté 1,6-bis(méthylpiperidinium)hexane dont la formule développée est donnée ci-dessous.

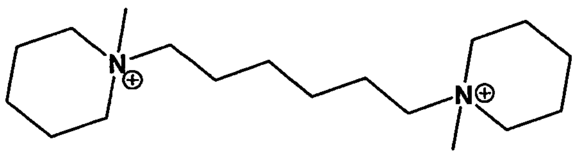

Les anions associés aux cations ammoniums quaternaires présents dans l'espèce organique structurante pour la synthèse de la zéolithe IZM-2 présente dans le catalyseur selon l'invention sont choisis parmi l'anion acétate, l'anion sulfate, l'anion carboxylate, l'anion tétrafluoroborate, les anions halogénures tels que le fluorure, le chlorure, le bromure, l'iodure, l'anion hydroxyde et une combinaison de plusieurs d'entre eux. De manière préférée, les anions associés aux cations ammoniums quaternaires présents dans l'espèce structurante pour la synthèse de la zéolithe IZM-2 sont choisis

parmi l'anion hydroxyde et l'anion bromure. Ladite espèce organique azotée utilisée comme agent structurant de la zéolithe IZM-2 est synthétisée par toute méthode connue de l'Homme du métier. Pour la synthèse du dibromure de 1,6-bis(méthylpiperidinium)hexane, on procède au mélange d'une mole de 1,6-dibromohexane et d'au moins 2 moles de N-méthylpiperidine dans l'éthanol. Généralement, le mélange est porté à reflux pendant une durée comprise entre 3 et 10 heures. Après filtration, précipitation au moyen d'un solvant éthéré tel que le diéthyéther puis recristallisation dans un mélange éthanol/éther, on obtient du dibromure de 1,6-bis(méthylpiperidinium)hexane. Le dihydroxyde de 1,6-bis (méthylpiperidinium)hexane est préférentiellement obtenu par traitement à température ambiante d'une solution aqueuse de dibromure de 1,6-bis(méthylpiperidinium)hexane par de l'oxyde d'argent $Ag_2O$.

La source de l'élément X, employée pour la mise en oeuvre du procédé de préparation de la zéolithe IZM-2, peut être tout composé comprenant l'élément X et pouvant libérer cet élément en solution aqueuse sous forme réactive. Avantageusement, lorsque l'élément X est le silicium, la source de silice peut être l'une quelconque de celles couramment utilisées dans la synthèse des zéolithes, par exemple de la silice solide en poudre, de l'acide silicique, de la silice colloïdale, de la silice dissoute ou du tétraéthoxysilane (TEOS). Parmi les silices en poudre, on peut utiliser les silices précipitées, notamment celles obtenues par précipitation à partir d'une solution de silicate de métal alcalin, telle que des silices aérosiles, des silices pyrogénées, par exemple du "CAB-O-SIL" et des gels de silice. On peut utiliser des silices colloïdales présentant différentes tailles de particules, par exemple de diamètre équivalent moyen compris entre 10 et 15 nm ou entre 40 et 50 nm telles que celles commercialisées sous les marques déposées telle que "LUDOX". De manière préférée, la source de silicium est le LUDOX AS-40.

La source de l'élément Y employée pour la mise en oeuvre du procédé de préparation de la zéolithe IZM-2 peut être tout composé comprenant l'élément Y et pouvant libérer cet élément en solution aqueuse sous forme réactive. Dans le cas préféré où Y est l'aluminium, la source d'alumine est de préférence de l'aluminate de sodium, ou un sel d'aluminium, par exemple du chlorure, du nitrate, de l'hydroxyde ou du sulfate, un alkoxyde d'aluminium ou de l'alumine proprement dite, de préférence sous forme hydratée ou hydratable, comme par exemple de l'alumine colloïdale, de la pseudoboehmite, de l'alumine gamma ou du trihydrate alpha ou bêta. On peut également utiliser des mélanges des sources citées ci-dessus.

Pour la source de métal M alcalin et/ou alcalino-terreux, on utilise avantageusement un halogénure ou un hydroxyde dudit métal M, de préférence un hydroxyde dudit métal M.

Pour la mise en oeuvre du procédé de préparation de la zéolithe IZM-2, il est préféré que le mélange aqueux, comportant au moins une source d'au moins un oxyde $XO_2$, au moins une source d'au moins un oxyde $Y_2O_3$, au moins une source d'au moins un métal alcalin et/ou alcalino-terreux, au moins une espèce organique R ayant deux atomes d'azote quaternaires, comporte également au moins une source d'ions hydroxydes. Ladite source d'ions hydroxydes provient avantageusement de l'espèce organique structurante R lorsqu'elle se trouve sous sa forme hydroxyde, à savoir le dihydroxyde de 1,6-bis(méthylpiperidinium)hexane, ou encore de la source de métal M alcalin et/ou alcalino-terreux lorsqu'il se trouve sous forme hydroxyde, par exemple l'hydroxyde de sodium.

Aussi, selon un mode de réalisation préféré du procédé de préparation de la zéolithe IZM-2 présente dans le catalyseur selon l'invention, on fait réagir un mélange aqueux comportant un oxyde de silicium, de l'alumine, du dibromure de 1,6-bis(méthylpiperidinium)hexane et de l'hydroxyde de sodium.

Le procédé de préparation de la zéolithe IZM-2 présente dans le catalyseur selon l'invention consiste à préparer un mélange réactionnel aqueux appelé gel et renfermant au moins une source d'au moins un oxyde $XO_2$, au moins une source d'au moins un oxyde $Y_2O3$, au moins une espèce organique R, au moins une source d'au moins un métal alcalin et/ou alcalino-terreux. Les quantités desdits réactifs sont ajustées de manière à conférer à ce gel une composition permettant sa cristallisation en zéolithe IZM-2 sous sa forme brute de synthèse de formule générale (I) $XO_2 : aY_2O_3 : bM_{2/n}O : cR : dH_2O$, où a, b, et n répondent aux critères définis plus haut, c représente le nombre de moles de R et est compris entre 0,005 et 2, de préférence entre 0,01 et 0,5 et d représente le nombre de moles de $H_2O$ et est compris entre 0,005 et 2 et de préférence entre 0,01 et 1. Puis le gel est soumis à un traitement hydrothermal jusqu'à ce que la zéolithe IZM-2 se forme. Le gel est avantageusement mis sous conditions hydrothermales sous une pression de réaction autogène, éventuellement en ajoutant du gaz, par exemple de l'azote, à une température comprise entre 120°C et 200°C, de préférence entre 140°C et 180°C, et de manière encore plus préférée entre 160 et 175°C jusqu'à la formation des cristaux de zéolithe IZM-2 sous sa forme brute de synthèse. La durée nécessaire pour obtenir la cristallisation varie généralement entre 1 heure et plusieurs mois en fonction de la composition des réactifs dans le gel, de l'agitation et de la température de réaction. De préférence la durée de cristallisation varie entre 2 heures et 21 jours. La mise en réaction s'effectue généralement sous agitation ou en absence d'agitation, de préférence en présence d'agitation.

Il peut être avantageux d'additionner des germes au mélange réactionnel afin de réduire le temps nécessaire à la formation des cristaux et/ou la durée totale de cristallisation. Il peut également être avantageux d'utiliser des germes afin de favoriser la formation de la zéolithe IZM-2 au détriment d'impuretés. De tels germes comprennent des solides cristallisés, notamment des cristaux de zéolithe IZM-2. Les germes cristallins sont généralement ajoutés dans une proportion comprise entre 0,01 et 10 % de la masse de l'oxyde $XO_2$ utilisée dans le mélange réactionnel.

A l'issue de l'étape de traitement hydrothermal conduisant à la cristallisation de la zéolithe IZM-2, la phase

solide est filtrée et lavée, on obtient ainsi la zéolithe IZM-2 sous sa forme brute de synthèse laquelle est séchée puis calcinée pour obtenir la zéolithe sous forme calcinée. L'étape de calcination s'effectue avantageusement par une ou plusieurs étapes de chauffage réalisée à une température comprise entre 100 et 1000°C, de préférence comprise entre 400 et 650°C, pour une durée comprise entre quelques heures et plusieurs jours, de préférence comprise entre 3 heures et 48 heures. De manière préférée, la calcination s'effectue en deux étapes de chauffage consécutives. A l'issue de ladite étape de calcination, la zéolithe IZM-2 obtenue est celle présentant le diagramme de diffraction de rayons X incluant au moins les raies inscrites dans le tableau 1. Elle est dépourvue d'eau ainsi que de l'espèce organique R présentes dans la zéolithe IZM-2 sous sa forme brute de synthèse.

L'invention concerne aussi la préparation dudit catalyseur. Pour préparer le catalyseur selon l'invention, on soumet généralement, dans un premier temps, ladite zéolithe IZM-2, soit sous sa forme brute de synthèse soit sous sa forme calcinée, à au moins une étape d'échange ionique par exemple par au moins une solution de $NH_4NO_3$ de manière à éliminer au moins en partie, de préférence pratiquement totalement, tout cation alcalin, en particulier le sodium, présent en position cationique dans la zéolithe et à obtenir ainsi ladite zéolithe IZM-2 sous forme hydrogène. Lorsque la(es)dite (s) étape(s) d'échange ionique est(sont) réalisée(s) sur une zéolithe IZM-2 brute de synthèse, on soumet généralement la zéolithe ainsi obtenue sous forme hydrogène à une étape de calcination sous flux d'air sec, qui a pour but d'éliminer le structurant organique occlus dans la microporosité de la zéolithe.

A ce stade, la zéolithe peut subir tout type de traitement connus de l'Homme du métier visant à la stabiliser, la désaluminer ou la passiver.

On poursuit la préparation du catalyseur en mélangeant la matrice et la zéolithe préparée précédemment puis on met en forme. La mise en forme du catalyseur selon l'invention est généralement telle que le catalyseur est de préférence sous forme d'extrudés ou de billes, en vue de son utilisation. Les conditions de mise en forme de la zéolithe, le choix de la matrice, éventuellement le broyage préalable de la zéolithe, le procédé de peptisation, l'ajout d'agents porogènes, le temps de malaxage, la pression d'extrusion si le catalyseur est mis sous forme d'extrudés, la vitesse et le temps de séchage, sont déterminés, pour chaque matrice, selon les règles bien connues de l'homme de métier, de manière à obtenir un catalyseur de préférence sous forme d'extrudés ou de billes.

La préparation du catalyseur se poursuit généralement par une calcination, habituellement à une température comprise entre 250°C et 600°C, de préférence précédée d'un séchage, par exemple à l'étuve, à une température généralement comprise entre la température ambiante et 250°C, de préférence entre 40°C et 200°C. Ladite étape de séchage est de préférence menée pendant la montée en température nécessaire pour effectuer ladite calcination.

La mise en forme de la zéolithe IZM-2 peut être effectuée sur la zéolithe brute de synthèse, c'est-à-dire contenant le structurant organique et des cations alcalins, généralement le sodium. Dans ce cas l'étape de calcination sous flux d'air sec, ayant pour but d'éliminer le structurant organique, et les étapes d'échanges ioniques par au moins une solution de $NH_4NO_3$ sont réalisées sur le catalyseur mis en forme comprenant la zéolithe et la matrice. Néanmoins et de manière très préférée, la(es) étape(s) d'échange(s) ionique(s), par exemple par au moins une solution de $NH_4NO_3$, réalisée(s) de manière à obtenir ladite zéolithe IZM-2 présente dans le catalyseur selon l'invention sous forme hydrogène est(sont) réalisée(s) avant mise en forme.

Le dépôt d'au moins un métal choisi parmi les métaux des groupes VIII, VIB et VIIB de la classification périodique des éléments, et éventuellement d'au moins un métal additionnel choisi parmi les métaux des groupes IIIA et IVA de la classification périodique des éléments, peut être effectué à tout moment de la préparation, soit avant la mise en forme, soit lors du mélange de la zéolithe et de la matrice, la zéolithe étant mélangée à l'ensemble constitué par le(s) précurseur (s) du(des)dit(s) métal(ux) et la matrice, soit, de manière préférée, après la mise en forme.

Lorsque l'ajout d'au moins un métal choisi parmi les métaux des groupes VIB, VIIB et VIII et éventuellement d'au moins un métal additionnel choisi parmi les métaux des groupes IIIA et IVA est effectué après la mise en forme, le(les)dit(s) métal(ux) peut(peuvent) alors être ajouté(s), soit avant la calcination, soit, de préférence, après la calcination du mélange matrice-zéolithe. Le(les)dit(s) métal(ux) ajouté(s) est(sont) généralement déposé(s), soit pratiquement totalement sur la zéolithe, soit en partie sur la zéolithe et en partie sur la matrice, soit, de préférence, pratiquement totalement sur la matrice, ceci s'effectuant, de la manière qui est connue de l'Homme du métier, par le choix approprié des paramètres utilisés lors dudit dépôt, comme par exemple la nature du précurseur du(des)dit(s) métal(ux). Le dépôt d'au moins un métal choisi parmi les métaux des groupes VIB, VIIB et VIII est généralement effectué par la technique d'imprégnation à sec, d'imprégnation par excès, ou de préférence par échange(s) ionique(s). Dans le cas de l'échange ionique à partir de précurseurs à base de platine et/ou de palladium, on utilise habituellement des sels de platine et/ou de palladium tels que l'acide hexachloroplatinique et/ou l'acide hexachloropalladique, en présence ou en absence d'agent (s) compétiteur(s), tel(s) que par exemple l'acide chlorhydrique. Dans le cas où au moins un métal additionnel choisi parmi les métaux des groupes IIIA et IVA de la classification périodique des éléments est également introduit, toutes les techniques de dépôt connues de l'Homme du métier et tous les précurseurs conviennent pour l'introduction dudit métal additionnel.

Dans le cas où le catalyseur contient plusieurs métaux choisis parmi les métaux des groupes VIB, VIIB et VIII de la classification périodique des éléments, les métaux peuvent être introduits, soit tous de la même façon, soit par

des techniques différentes, et dans n'importe quel ordre. Dans le cas où au moins un métal choisi parmi les métaux des groupes IIIA et IVA de la classification périodique des éléments est également introduit, on peut ajouter les métaux choisis parmi les métaux des groupes VIB, VIIB et VIII et ceux choisis parmi les métaux des groupes IIIA ou IVA soit séparément soit simultanément dans au moins une étape unitaire. Lorsqu'au moins un métal choisi parmi les métaux des groupes IIIA et IVA est ajouté séparément, il est préférable qu'il soit ajouté préalablement au(x) métal(ux) choisis parmi les métaux des groupes VIB, VIIB et VIII. Dans le cas où la technique de dépôt utilisée est celle de l'échange ionique, plusieurs échanges successifs peuvent être nécessaires pour introduire les quantités requises de métaux.

Le(s) métal(ux) choisi(s) parmi les métaux des groupes VIB, VIIB et VIII est(sont) avantageusement déposé (s) dans la matrice sous forme d'acide de composés ammoniaqués ou de composés tels que par exemple les nitrates et les chlorures. De préférence pour le platine, on choisira l'acide hexachloroplatinque ou des sels de platine tetramines. Pour le Re, l'acide perhenique est préféré. Pour le Ni, le nitrate de Ni est préféré. Pour le Mo, l'heptamolybdate d'ammonium est préféré.

De manière préférée, lorsque le catalyseur selon l'invention comprend au moins un métal noble, par exemple le platine ou le palladium, on utilise avantageusement comme précurseur des composés ammoniaqués. Dans ce cas, le métal noble sera déposé sur la zéolithe.

Dans le cas du platine, on peut citer par exemple les sels de platine II tétramines de formule $Pt(NH_3)_4X_2$, les sels de platine IV hexamines de formule $Pt(NH_3)_6X_4$; les sels de platine IV halogénopentamines de formule $(PtX(NH_3)_5)X_3$ ; les sels de platine N-tétrahalogénodiamines de formule $PtX_4(NH_3)_2$ ; les complexes de platine avec les halogènes-polycétones et les composés halogénés de formule $H(Pt(acac)_2X)$ ; X étant un halogène choisi dans le groupe formé par le chlore, le fluor, le brome et l'iode, et de préférence X étant le chlore, et acac représentant le groupe $C_5H_7O_2$ dérivé de l'acétylacétone.

L'introduction d'au moins un métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Le métal additionnel, éventuellement introduit en plus, choisi parmi les métaux des groupes IIIA et IVA, peut être introduit par l'intermédiaire de composés tels que par exemple les chlorures, les bromures et les nitrates, les alkyls des métaux des groupes IIIA et IVA, soit par exemple pour l'étain et l'indium, les alkyl étain, le nitrate et le chlorure d'indium.

Si ledit métal additionnel est introduit avant le métal noble, le composé dudit métal additionnel utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain dans le cas de l'étain. Dans ce cas, avant de procéder à l'introduction d'au moins un métal noble, on procède à une calcination sous air.

Ledit métal additionnel peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés du métal additionnel, on peut citer en particulier le tétrabutylétain dans le cas de l'étain, et le triphénylindium dans le cas de l'indium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Le dépôt d'au moins un métal choisi parmi les métaux des groupes VIB, VIIB et VIII et éventuellement d'au moins un métal choisi parmi les métaux des groupes IIIA et IVA est suivi de préférence d'une calcination sous air ou oxygène, généralement entre 250° C et 600°C, de préférence entre 350°C et 550°C, et pour une durée comprise entre 0,5 et 10 heure(s), de préférence entre 1 et 4 heure(s). On procède ensuite éventuellement à une réduction sous hydrogène, généralement à une température comprise entre 300 et 600°C, de préférence entre 350°C et 550°C, et pour une durée comprise entre 1 et 10 heure(s), de préférence entre 2 et 5 heures, de façon à obtenir le(les)dit(s) métal(ux) principalement sous forme réduite nécessaire à l'activité catalytique.

Par exemple, une des méthodes préférées de préparation du catalyseur selon l'invention consiste d'abord à soumettre ladite zéolithe IZM-2, soit sous sa forme brute de synthèse soit sous sa forme calcinée, à au moins une étape d'échange ionique, par exemple avec au moins une solution de $NH_4NO_3$ de manière à obtenir ladite zéolithe sous sa forme hydrogène. Ladite zéolithe IZM-2 ainsi échangée est ensuite malaxée dans un gel humide de matrice (obtenu généralement par mélange d'au moins un acide et d'une poudre de matrice), par exemple d'alumine, pendant une durée nécessaire pour l'obtention d'une bonne homogénéité de la pâte ainsi obtenue, soit par exemple pendant une dizaine

de minutes, puis à passer ladite pâte à travers une filière pour former des extrudés, par exemple de diamètre compris entre 0,4 et 4 mm, de préférence entre 0,4 et 2,5 mm et de préférence encore entre 0,8 et 2,0 mm. Puis, après séchage et calcination, le ou les métal(ux) choisi(s) parmi les métaux des groupes VIB, VIIB et VIII, par exemple le platine, et éventuellement le ou les métal(ux) choisi(s) parmi les métaux des groupes IIIA et IVA, est(sont) déposé(s), par exemple par échange ionique, avec par exemple de l'acide hexachloroplatinique en présence d'un agent compétiteur (par exemple l'acide chlorhydrique), ledit dépôt étant suivi d'une calcination, par exemple pendant environ 2 heures à environ 400°C.

Quel que soit le mode de réalisation de la préparation du catalyseur selon l'invention, on peut mettre en oeuvre une réduction préalable du catalyseur selon l'invention ex *situ,* sous courant d'hydrogène, par exemple à une température de 450°C à 600°C, pendant une durée de 0,5 à 4 heures.

Dans le cas où le catalyseur ne contient pas de soufre, une réduction du métal sous hydrogène est réalisée *in situ* avant injection de la charge.

Dans le cas où le catalyseur de l'invention contient du soufre, le soufre est introduit sur le catalyseur mis en forme, calciné, contenant le ou les métaux cités précédemment, soit *in situ* avant la réaction catalytique, soit *ex situ.* La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration ex *situ,* on effectue la réduction puis la sulfuration. La sulfuration s'effectue en présence d'hydrogène en utilisant tout agent sulfurant bien connu de l'Homme du métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

La présente invention a également pour objet des procédés de transformation d'hydrocarbures en présence d'au moins dudit catalyseur selon l'invention. Les transformations d'hydrocarbures selon l'invention concernent notamment l'isomérisation des composés aromatiques à 8 atomes de carbone, la transalkylation des composés alkylaromatiques, l'hydroisomérisation de paraffines linéaires légères et la transformation d'alcools.

Plus précisément, un autre objet de la présente invention est un procédé d'isomérisation d'une coupe contenant au moins un composé aromatique à huit atomes de carbone par molécule, ledit procédé comprenant la mise en contact de ladite coupe aromatique avec au moins ledit catalyseur selon l'invention présent dans au moins un réacteur catalytique. Ladite coupe aromatique contenant au moins un composé aromatique ayant huit atomes de carbone par molécule comprend en particulier comme composé aromatique ayant huit atomes de carbone par molécule soit uniquement un mélange de xylènes, soit uniquement de l'éthylbenzène, soit un mélange de xylène(s) et d'éthylbenzène. Ledit procédé d'isomérisation est mis en oeuvre généralement selon les conditions opératoires suivantes :

- une température de 300°C à 500°C, de préférence de 320°C à 450°C et de manière encore plus préférée de 340°C à 430°C ;
- une pression partielle d'hydrogène de 0,3 à 1,5 MPa, de préférence de 0,4 à 1,2 MPa et de manière encore préférée de 0,7 à 1,2 MPa ;
- une pression totale de 0,45 à 1,9 MPa, de préférence de 0,6 à 1,5 MPa ; et
- une vitesse spatiale d'alimentation, exprimée en kilogramme de charge introduite par kilogramme de catalyseur et par heure, de 0,25 à 30 $h^{-1}$, de préférence de 1 à 10 $h^{-1}$ et de manière encore préférée de 2 à 6 $h^{-1}$.

De préférence, le catalyseur utilisé pour la mise en oeuvre dudit procédé d'isomérisation selon l'invention comprend au moins une zéolithe IZM-2, au moins une matrice et au moins un métal du groupe VIII, de préférence le platine ou le palladium. De manière très avantageuse, il comprend également au moins un métal additionnel choisi parmi les métaux des groupes IIIA et IVA. Il peut également comprendre une teneur en soufre telle que le rapport du nombre d'atomes de soufre sur le nombre d'atomes de métal(ux) du groupe VIII soit compris entre 0,5 : 1 et 2 : 1.

Un autre objet de la présente invention est un procédé de transalkylation d'hydrocarbures alkylaromatiques pour produire des xylènes, ledit procédé comprenant la mise en contact desdits hydrocarbures alkylaromatiques avec au moins ledit catalyseur selon l'invention présent dans au moins un réacteur catalytique. De préférence, le procédé de transalkylation selon l'invention est un procédé de transalkylation du toluène et d'hydrocarbures alkylaromatiques contenant au moins 9 atomes de carbone par molécule ($AC_9$+), de préférence des triméthylbenzènes, pour produire des xylènes. La charge utilisée pour la mise en oeuvre dudit procédé de transalkylation selon l'invention est généralement formée d'un mélange toluène - $AC_9^+$ pouvant contenir de 0,1 à 100% poids de $AC_9^+$ par rapport au mélange total. Ledit catalyseur selon l'invention se révèle très efficace pour ladite utilisation, car il se révèle être particulièrement actif, sélectif et stable, même en présence de charges à traiter contenant une grande quantité d'aromatiques lourds $AC_9^+$, ces aromatiques lourds pouvant contenir une grande proportion d'$AC_{10}^+$. Ainsi, des charges $AC_9^+$ contenant au moins 5 % et jusqu'à 25 % poids, et même davantage d'$AC_{10}^+$ peuvent être valorisées. A titre d'exemples, on peut citer de manière non exhaustive, les diméthyléthylbenzènes, les diéthylbenzènes, les propyléthylbenzène. L'utilisation dudit catalyseur selon l'invention en transalkylation d'alkylaromatiques lourds est donc particulièrement intéressante.

Les conditions opératoires pour la mise en oeuvre du procédé de transalkylation d'hydrocarbures alkylaromatiques selon l'invention sont généralement les suivantes : une température comprise entre 250 et 650°C et de préférence entre 350 et 550°C ; une pression comprise entre 1 et 6 MPa et de préférence entre 2 et 4,5 MPa ; une vitesse spatiale d'alimentation, exprimée en kilogramme de charge introduite par kilogramme de catalyseur et par heure, comprise entre 0,1 et 10 $h^{-1}$ et de préférence entre 0,5 et 4 $h^{-1}$ ; un rapport molaire hydrogène sur hydrocarbures compris entre 2 et 20 et de préférence entre 3 et 12 mol/mol.

De préférence, le catalyseur utilisé pour la transalkylation d'hydrocarbures alkylaromatiques comprend au moins une zéolithe IZM-2, au moins une matrice aluminique et au moins un métal du groupe VIIB, de préférence le rhénium.

Un autre objet de la présente invention est un procédé d'hydroisomérisation des paraffines présentes dans une charge comprenant en majeure partie des paraffines linéaires contenant de 5 à 8 atomes de carbone par molécule, ledit procédé comprenant la mise en contact de ladite charge avec au moins ledit catalyseur selon l'invention. De manière préférée, la somme des teneurs en paraffines linéaires à 7 et à 8 atomes de carbone par molécule contenues dans la charge est comprise entre 2 et 90 % poids, de préférence entre 5 et 90 % poids, de manière plus préférée entre 20 et 90 % poids, et de façon très préférée entre 40 et 90 % poids par rapport à la charge. Ladite charge est traitée dans au moins une zone réactionnelle contenant au moins ledit catalyseur selon l'invention disposé de préférence en lit fixe.
Les conditions opératoires pour la mise en oeuvre dudit procédé d'hydroisomérisation selon l'invention sont généralement les suivantes :

- une température de 30°C à 300°C, de préférence de 70°C à 300°C et de manière encore plus préférée de 80°C à 280°C ;
- une pression totale de 0,10 à 18 MPa, de préférence de 0,5 à 10 MPa ; et de manière encore plus préférée de 2 à 5 MPa ;
- une vitesse volumique horaire (pph) définie comme la masse de charge à traiter par masse de catalyseur et par heure comprise entre 0,2 et 10 $h^{-1}$, de préférence comprise entre 0,3 et 5 $h^{-1}$ et d'une manière encore plus préférée comprise entre 0,5 et 2 $h^{-1}$.
- rapport molaire H2/HC compris entre 0,05 et 20, de préférence entre 0,2 et 10.

De préférence, le catalyseur utilisé pour l'hydroisomérisation des paraffines comprend au moins une zéolithe IZM-2, au moins une matrice aluminique et au moins un métal du groupe VIII, de préférence le platine ou le palladium.

Un autre objet de l'invention porte sur un procédé de transformation d'au moins un composé aliphatique ayant de 1 à 18 atomes de carbone et portant une fonction alcool, ledit procédé étant réalisé en présence d'au moins un catalyseur selon l'invention.
De manière préférée, ledit composé aliphatique portant une fonction alcool comprend de 1 à 12 atomes de carbone et de manière plus préférée de 1 à 6 atomes de carbone. De manière encore plus préférée, ledit composé aliphatique portant une fonction alcool est choisi parmi l'éthanol et le pentanol. Ledit composé aliphatique ayant de 1 à 18 atomes de carbone et portant une fonction alcool peut être linéaire ou ramifié. De préférence, il s'agit d'un monoalcool. L'utilisation d'alcools totalement anhydres n'est pas nécessaire pour la mise en oeuvre dudit procédé de transformation d'au moins un composé aliphatique ayant de 1 à 18 atomes de carbone et portant une fonction alcool.
Selon un premier mode de réalisation dudit procédé de transformation d'au moins un composé aliphatique ayant de 1 à 18 atomes de carbone et portant une fonction alcool, ladite transformation mise en jeu est une réaction de déshydratation au cours de laquelle ledit composé aliphatique portant une fonction alcool est déshydraté en oléfine(s) avec production d'eau. Conformément audit premier mode, on utilise préférentiellement l'éthanol comme composé aliphatique ayant une fonction alcool de manière à produire de l'éthylène. Les conditions opératoires pour la mise en oeuvre dudit procédé de transformation des alcools en oléfines sont les suivantes : la pression totale est inférieure à 2 MPa, de préférence comprise entre 0,05 et 1 MPa, la température est comprise entre 150 et 400 °C, de préférence comprise entre 200 et 300°C. La pph définie comme étant le débit massique d'introduction de la charge comprenant ledit composé aliphatique divisé par la masse de catalyseur dépend alors de l'alcool présent dans cette charge et varie en général entre 0,5 et 50 $h^{-1}$ et préférentiellement entre 1 et 25 $h^{-1}$. Un gaz inerte comme par exemple de l'azote ou un hydrocarbure léger peut être utilisé pour diluer la charge comprenant ledit composé aliphatique au niveau du catalyseur.
Ledit procédé de transformation des alcools en oléfines conformément audit premier mode de réalisation est avantageusement mis en oeuvre en lit fixe, mobile ou fluidisé. Hormis l'eau générée lors de la réaction de déshydratation, les éthers associés aux alcools introduits dans le réacteur peuvent être formés principalement dans le cas du méthanol et de l'éthanol. Lesdits éthers peuvent être avantageusement recyclés afin d'augmenter le rendement en oléfines.

Selon un deuxième mode de réalisation dudit procédé de transformation d'au moins un composé aliphatique ayant de 1 à 18 atomes de carbone et portant une fonction alcool, ladite transformation mise en jeu réalise simultanément dans un même réacteur la déshydratation dudit composé aliphatique en oléfine(s) et l'oligomérisation de(s)dite(s) oléfine (s). Il s'agit de produire des hydrocarbures qui seront incorporés dans le pool essence et / ou dans le pool diesel. Les

conditions opératoires pour la mise en oeuvre d'une telle transformation sont telles que la température est comprise entre 250°C et 450°C, la pression totale est comprise entre 2 et 10 MPa et la PPH correspondant au débit massique d'introduction de la charge comprenant ledit composé aliphatique divisé par la masse de catalyseur est comprise entre 0,1 et 5 $h^{-1}$. L'augmentation de la pression pour la mise en oeuvre dudit deuxième mode par rapport audit premier mode de réalisation (déshydratation) favorise la formation de composés issus de l'oligomérisation des oléfines formées *in situ* dans le(s) réacteur(s). Le catalyseur à base d'IZM-2 selon l'invention est préférentiellement activé, de préférence en le soumettant à une calcination, préalablement à sa mise en contact dans le réacteur avec la charge comprenant ledit composé aliphatique aux conditions de réaction précitées. Un gaz inerte tel que l'azote ou un hydrocarbure léger est avantageusement utilisé pour diluer la charge au niveau du catalyseur.
Une variante dudit deuxième mode de réalisation du procédé de transformation selon l'invention consiste à séparer la mise en oeuvre de l'étape de déshydratation de celle de l'oligomérisation des oléfines formées dans l'étape de déshydratation. Conformément à cette variante, un séparateur est avantageusement installé entre le réacteur utilisé pour la déshydratation des alcools en oléfines et le réacteur utilisé pour la transformation des oléfines en composés plus lourds. On peut réaliser la réaction de déshydratation et la réaction d'oligomérisation en présence d'un catalyseur à base d'une zéolithe IZM-2 selon l'invention ou réaliser la réaction de déshydratation en présence d'un catalyseur comprenant une zéolithe différente de la zéolithe IZM-2, une silice-alumine ou une alumine activée et réaliser la réaction d'oligomérisation en présence d'un catalyseur à base d'une zéolithe IZM-2 selon l'invention.
Quel que soit le mode de réalisation mis en oeuvre pour la transformation d'une charge comportant au moins un composé aliphatique portant une fonction alcool, la réaction de transformation de ladite charge par déshydratation ou par déshydratation puis oligomérisation peut-être réalisée dans tout type de réacteur connu de l'Homme du métier. Selon une première mise en oeuvre, ledit procédé de transformation de ladite charge est réalisé dans au moins un réacteur en lit fixe. Le catalyseur se situe alors préférentiellement dans un réacteur à lit radial afin de minimiser la perte de charge au travers du lit catalytique. Selon une deuxième mise en oeuvre, ledit procédé de transformation de ladite charge est réalisé dans au moins un réacteur en lit mobile. On peut utiliser un ou plusieurs réacteurs avec un ou plusieurs lits mobiles, avec injection étagée possible de la charge, couplés ou non à un système de régénération en continu.
Conformément audit procédé de transformation d'une charge comportant au moins un composé aliphatique portant une fonction alcool selon l'invention, l'effluent de réaction est conservé à sa pression de réaction, aux pertes de charge dans les équipements traversés près. L'effluent est refroidi en dessous du point de rosée de l'eau. S'agissant dudit deuxième mode de réalisation (déshydratation + oligomérisation), ledit effluent de réaction refroidi est introduit dans un dispositif permettant la séparation triphasique d'une phase gazeuse constituée notamment d'oléfines légères, d'un liquide organique (essence et gazole) et d'un liquide aqueux (eau, alcool non transformé, hydrocarbures solubilisés).
Les exemples qui suivent illustrent l'invention sans en limiter la portée.

Exemple 1 : préparation du dibromure de 1,6-bis(méthylpiperidinium)hexane pour la préparation des zéolithes IZM-2 (Z1 et Z2).

50 g de 1,6-dibromohexane (0,20 mole, 99%, Alfa Aesar) sont ajoutés dans un ballon de 1 L contenant 50 g de N-méthylpipéridine (0,51 mole, 99%, alfa Aesar) et 200 ml d'éthanol. Le milieu réactionnel est agité et porté à reflux pendant 5 h. Le mélange est ensuite refroidi à température ambiante puis filtré. Le mélange est versé dans 300 ml de diéthyléther froid puis le précipité formé est filtré et lavé avec 100 ml de diéthyléther. Le solide obtenu est recristallisé dans un mélange éthanol/éther. Le solide obtenu est séché sous vide pendant 12 h. On obtient 71 grammes d'un solide blanc (soit un rendement de 80 %). Le produit obtenu possède le spectre 1 H RMN attendu. 1H RMN ($D_2O$ ppm/TMS) : 1,27 (4H,m) ; 1,48 (4H,m) ; 1,61 (4H,m) ; 1,70 (8H,m) ; 2,85 (6H,s) ; 3,16 (12H,m).

Exemple 2 : préparation d'une zéolithe IZM-2 sous sa forme hydrogène

20,134 g d'une suspension colloïdale de silice, connue sous le terme commercial Ludox HS-40 commercialisée par Aldrich, est incorporée dans une solution composée de 0,253 g d'aluminate de sodium (carlo erba), de 1,555 g de soude (prolabo), 9,888 g de 1,6-bis(méthylpiperidinium)hexane et de 68,170 g d'eau déionisée. La composition molaire du mélange est la suivante : $SiO_2$ ; 0,01 $Al_2O_3$ ; 0,17 $Na_2O$ ; 0,17 1,6-bis(méthylpiperidinium)hexane ; 33,33 $H_2O$. Le mélange est agité vigoureusement pendant une demi-heure. Le mélange est ensuite transféré, après homogénéisation, dans un autoclave. L'autoclave est chauffé pendant 9 jours à 170°C sous agitation (250 tours/min). Le produit cristallisé obtenu est filtré, lavé à l'eau déionisée (pour atteindre un pH neutre) puis séché une nuit à 100°C. Le solide est ensuite introduit dans un four à moufle où est réalisée la calcination : le cycle de calcination comprend une montée en température jusqu'à 200°C, un palier à 200°C maintenu durant 2 heures, une montée en température jusqu'à 550°C suivi d'un palier à 550°C maintenu durant 8 heures puis un retour à la température ambiante.
Le solide calciné a été analysé par diffraction des rayons X et identifié comme étant constitué de zéolithe IZM-2.
Ladite zéolithe IZM-2 est ensuite mise en contact pendant 2 heures à température ambiante avec une solution

aqueuse à 1 mole de chlorure d'ammonium en utilisant 50 ml de solution par gramme de produit calciné solide. La zéolithe a ensuite été filtrée, lavée à l'eau permutée et séchée à 110°C. Ce traitement a été répété 3 fois. La zéolithe a ensuite été calcinée sous air pendant 24 heures, à 550°C. Elle se trouve sous forme hydrogène.

Exemple 3 : Préparation d'un catalyseur C1 comprenant une zéolithe IZM-2, du chrome et une matrice aluminique (invention)

La zéolithe IZM-2 sous forme hydrogène, préparée selon l'exemple 2, est soumise à une étape d'imprégnation à sec par une solution aqueuse de sulfate de chrome. Le produit est ensuite séché à 110°C pendant 18 heures puis calciné sous air pendant 12 heures à 550°C. La teneur pondérale en chrome du produit constitué de la zéolithe IZM-2 sous forme hydrogène et du chrome est de 0,21 %. Ledit produit est malaxé avec un gel d'alumine de type SB3 fourni par la société Sasol. La pâte malaxée est alors extrudée au travers d'une filière de diamètre 1,4 mm. Les extrudés ainsi obtenus sont calcinés à 500°C pendant 2 heures sous air. On obtient ainsi le catalyseur C1 constitué de 40% poids d'alumine, de 59,87% poids de zéolithe IZM-2 et de 0,13 % poids de $Cr_2O_7$. Ladite zéolithe IZM-2 présente une composition chimique de formule $SiO_2$ : 0,0125 $Al_2O_3$ (a = 0,0125) soit un rapport Si/Al égal à 40.

Exemple 4 : Préparation d'un catalyseur C2 comprenant une zéolithe IZM-2 (forme H)' du nickel et une matrice aluminique (invention)

La zéolithe IZM-2 sous forme hydrogène et préparée selon l'exemple 2 est soumise à une étape d'imprégnation à sec par une solution aqueuse de sulfate de nickel. Le produit est ensuite séché à 110°C pendant 18 heures puis calciné sous air pendant 12 heures à 550°C. La teneur pondérale en nickel du produit constitué de la zéolithe IZM-2 sous forme hydrogène et du nickel est de 0,23%. Ledit produit est malaxé avec un gel d'alumine de type SB3 fourni par la société Sasol. La pâte malaxée est alors extrudée au travers d'une filière de diamètre 1,4 mm. Les extrudés ainsi obtenus sont calcinés à 500°C pendant 2 heures sous air. On obtient ainsi le catalyseur C2 constitué de 40% poids d'alumine, de 59,86% poids de zéolithe IZM-2 et de 0,14% poids de NiO. Ladite zéolithe IZM-2 présente une composition chimique de formule $SiO_2$ : 0,0125 $Al_2O_3$ (a = 0,0125) soit un rapport Si/Al égal à 40.

Exemple 5 : transformation d'alcools sous pression (invention)

On procède dans cet exemple à l'évaluation successive des performances des catalyseurs C1 et C2 dans la transformation de l'éthanol sous pression au cours de deux tests différents.
Pour chacun des tests, une unité pilote en lit fixe traversé est chargée avec 1,5 g de catalyseur C1 respectivement C2. Avant de procéder à chacun des tests, les catalyseurs C1 et C2 sont activés à 550°C sous air durant 2 h.
Pour la mise en oeuvre de chacun des tests, on dilue l'éthanol avec de l'azote de sorte que le rapport molaire $N_2$/éthanol soit égal à 4. A la sortie du réacteur, on procède à la séparation de la phase gaz, de la phase liquide organique et de la phase liquide aqueuse. Dans le tableau 2 sont indiqués, en plus des conditions expérimentales, le bilan massique des produits organiques récupérés. Ceux-ci sont divisés en trois catégories, les gaz, les liquides de faibles points d'ébullition (bp (boiling point) < 150°C) et les liquides de points d'ébullition élevés (bp > 150°C).

Tableau 2 : Conditions opératoires et performances des catalyseurs C1 et C2 dans la transformation de l'éthanol sous pression

| catalyseur | | C1 | C2 |
|---|---|---|---|
| | | | |
| Conditions | | | |
| | T (°C) | 300 | 300 |
| | P (MPa) | 3 | 3 |
| | pph ($h^{-1}$) | 1,2 | 1,2 |
| | *TOS(h) | 5 | 5 |
| | | | |
| Conversion éthanol | | 100% | 100,0% |
| | | | |

(suite)

| | | | |
|---|---|---|---|
| Répartition des produits dans l'effluent de sortie (% massique) | | | |
| | gaz | 22,1% | 20,2% |
| | liquide (bp<150°C) | 38,5% | 35,8% |
| | liquide (bp>150°C) | 39,4% | 44% |
| * TOS (time on stream) représente le temps de contact du catalyseur avec la charge | | | |

La conversion est calculée comme suit:

$$\text{Conversion} = (\text{débit massique d'alcool}_{\text{entrée}} - \text{débit massique d'alcool}_{\text{sortie}}) / \text{débit massique d'alcool}_{\text{entrée}}$$

Les résultats figurant dans le tableau 2 démontrent que les catalyseurs C1 et C2 selon l'invention sont très actifs dans la transformation d'alcools sous pression et conduisent à des produits aisément incorporables dans le pool essence (phase liquide ayant un point d'ébullition inférieur à 150°C) et dans le pool diesel (phase liquide ayant un point d'ébullition supérieur à 150°C).

Exemple 6 : Préparation du catalyseur C3 comprenant une zéolithe IZM-2 (forme H), du platine et une matrice aluminique (invention)

20,138 g d'une suspension colloïdale de silice, connue sous le terme commercial Ludox HS-40 commercialisée par Aldrich, est incorporée dans une solution composée de 0,211 g d'aluminate de sodium (carlo erba), de 1,577 g de soude (prolabo), 9,890 g de 1,6-bis(méthylpiperidinium)hexane et de 68,252 g d'eau déionisée. La composition molaire du mélange est la suivante : $SiO_2$ ; 0,008 $Al_2O_3$ ; 0,17 $Na_2O$ ; 0,17 1,6-bis(méthylpiperidinium)hexane ; 33,33 $H_2O$. Le mélange est agité vigoureusement pendant une demi-heure. Le mélange est ensuite transféré, après homogénéisation, dans un autoclave. L'autoclave est chauffé pendant 9 jours à 170°C sous agitation (250 tours/min). Le produit cristallisé obtenu est filtré, lavé à l'eau déionisée (pour atteindre un pH neutre) puis séché une nuit à 100°C. La zéolithe IZM-2 brute de synthèse ainsi obtenue a été analysée par diffraction des rayons X et identifiée comme étant constitué de zéolithe IZM-2.

Ladite zéolithe IZM-2 sous sa forme brute de synthèse est soumise à quatre échanges ioniques dans une solution de $NH_4NO_3$ 10N, à environ 100°C pendant 4 heures pour chaque échange de manière à obtenir ladite zéolithe IZM-2 sous forme hydrogène. Elle est ensuite mise en forme par extrusion avec un gel d'alumine. Les extrudés obtenus subissent une calcination dite sèche à 550°C sous flux d'air sec durant 10 heures de manière à éliminer le structurant organique. 1% poids de platine est déposé sur lesdits extrudés par imprégnation à sec à partir de $Pt(NH_3)_4Cl_2$. Après mise à l'étuve (110°C, 12h) et calcination sous air (21/h/g) à 420°C, on obtient le catalyseur C3 constitué de 89,10 % poids d'alumine, 9,90 % poids de zéolithe IZM-2 et 1 % poids de platine. Ladite zéolithe IZM-2 présente une composition chimique de formule $SiO_2$ : 0,0094 $Al_2O_3$ (a = 0,0094) soit un rapport Si/Al égal à 53.

Exemple 7 : Évaluation des propriétés catalytiques du catalyseur C3 en isomérisation des composés aromatiques à 8 atomes de carbone (invention)

Les performances du catalyseur C3 ont été évaluées dans l'isomérisation d'une charge aromatique constituée uniquement d'éthylbenzène.

Les conditions opératoires de l'isomérisation sont les suivantes :

- température: 410°C ;
- pression totale : 1 MPa ;
- pression partielle d'hydrogène : 0,8 MPa.
- vitesse spatiale d'alimentation, exprimée en kilogramme de charge introduite par kilogramme de catalyseur et par heure, égale à 8,7 $h^{-1}$.

Le catalyseur est introduit dans la zone réactionnelle où il est d'abord réduit sous hydrogène pendant 4 heures

à 480°C puis la charge est introduite dans ladite zone réactionnelle où elle est mise en contact avec ledit catalyseur C3. Le catalyseur a été évalué en termes de conversion d'éthylbenzène et de sélectivité en xylènes. Les résultats figurent dans le tableau 3.

Le rendement en xylènes est déterminé à partir du % massique des xylènes produits, calculé à partir des données obtenues par analyse chromatographique de chaque effluent.
La conversion de l'éthylbenzène est le pourcentage poids d'éthylbenzène consommé. La sélectivité en xylènes est calculée au moyen du rendement en xylènes produits. La sélectivité est égal au rapport du rendement en xylènes sur la conversion en éthylbenzène.

Tableau 3 : performances du catalyseur C3 dans l'isomérisation des composés aromatiques à 8 atomes de carbone après 4000 min de réaction.

| Catalyseur | C3 |
|---|---|
| conversion éthylbenzène(%) | 33,5 |
| sélectivité en xylènes (%) | 62,1 |
| Rendement en xylènes (%) | 20,8 |

<u>Exemple 8 : Préparation du catalyseur C4 comprenant une zéolithe IZM-2 (forme H), du rhénium et une matrice aluminique (invention)</u>

On prépare une zéolithe IZM-2 brute de synthèse selon un même protocole et dans les mêmes conditions opératoires (quantité des réactifs et conditions opératoires proprement dites) que celui et celles donnés pour la préparation de la zéolithe IZM-2 brute de synthèse préparée dans l'exemple 6.

Ladite zéolithe IZM-2 sous sa forme brute de synthèse est soumise à quatre échanges ioniques dans une solution de $NH_4NO_3$ 10N, à environ 100°C pendant 4 heures pour chaque échange de manière à obtenir ladite zéolithe IZM-2 sous forme hydrogène. Elle est ensuite mise en forme par extrusion avec un gel d'alumine. Les extrudés obtenus subissent une calcination dite sèche à 550°C sous flux d'air sec durant 10 heures de manière à éliminer le structurant organique. Ils sont ensuite imprégnés à l'aide d'une solution aqueuse de perrhénate d'ammonium de manière à déposer 0,3 % poids de rhénium sur le catalyseur final. Le solide humide est ensuite séché à 120°C pendant 12 heures et calciné sous un débit d'air sec à la température de 500°C pendant une heure. Le catalyseur C4 ainsi obtenu contient en poids 79,7 % de zéolithe IZM-2, 20,0 % d'alumine et 0,3 % de Re. Ladite zéolithe IZM-2 présente une composition chimique de formule $SiO_2$ : 0,0094 $Al_2O_3$ (a = 0,0094) soit un rapport Si/Al égale à 53.

<u>Exemple 9 : Performances catalytiques du catalyseur C4 en transalkylation d'aromatiques (invention)</u>

Le catalyseur C4 est d'abord réduit sous hydrogène à 450°C pendant 2 heures. Il est ensuite traité avec une charge contenant du disulfure de diméthyle (DMDS), avec une concentration telle que le rapport atomique soufre/métal soit de 1,5. Ce traitement est effectué pendant 3 heures à 400°C, en maintenant un rapport hydrogène/hydrocarbure égal à 4.
Le test catalytique a été réalisé dans les conditions opératoires suivantes :

- température : 400°C
- pression totale : 3 MPa
- H2/HC : 5 mol/mol.
- pph : $4h^{-1}$ (masse de charge par g de catalyseur et par heure).

Le catalyseur C4 a été évalué avec une charge contenant 50 % poids de toluène et 50 poids % d'une charge A1 constituée de 32% poids d'éthyltoluène, 56% poids de triméthylbenzène et 12% poids d'aromatiques à au moins 10 atomes de carbone. Les résultats sont présentés dans le tableau 4.

La conversion globale est le pourcentage massique de charge consommée (50% poids toluène + 50% poids charge AC9+ consommés).
Le rendement en produits de la réaction est déterminé à partir du % massique des produits, calculé à partir des données obtenues par analyse chromatographique de chaque effluent.

Tableau 4 : performances du catalyseur C4 en transalkylation de composés aromatiques

| | |
|---|---|
| Conversion globale (%) | 52,8 |

(suite)

| Rendements (%poids) | |
|---|---|
| Légers ($C_1$-$C_4$) | 10,1 |
| Benzène+xylènes | 42,8 |
| Ethylbenzène | 0,4 |
| lourds | 1,9 |

Le catalyseur C4 selon l'invention est actif dans le procédé de transalkylation des composés aromatiques à au moins 9 atomes de carbone par molécule et conduit à un rendement (benzène + xylènes) satisfaisant.

Exemple 10 : préparation d'un catalyseur C5 comprenant une zéolithe IZM-2 (forme H), du platine et une matrice aluminique (invention)

20,144 g d'une suspension colloïdale de silice, connue sous le terme commercial Ludox HS-40 commercialisée par Aldrich, est incorporée dans une solution composée de 0,158 g d'aluminate de sodium (carlo erba), de 1,604 g de soude (prolabo), 9,893 g de 1,6-bis(méthylpiperidinium)hexane et de 68,200 g d'eau déionisée. La composition molaire du mélange est la suivante : $SiO_2$ ; 0,006 $Al_2O_3$ ; 0,17 $Na_2O$ ; 0,17 1,6-bis(méthylpiperidinium)hexane ; 33,33 $H_2O$. Le mélange est agité vigoureusement pendant une demi-heure. Le mélange est ensuite transféré, après homogénéisation, dans un autoclave. L'autoclave est chauffé pendant 9 jours à 170°C sous agitation (250 tours/min). Le produit cristallisé obtenu est filtré, lavé à l'eau déionisée (pour atteindre un pH neutre) puis séché une nuit à 100°C. La zéolithe IZM-2 brute de synthèse ainsi obtenue a été analysée par diffraction des rayons X et identifiée comme étant constituée de zéolithe IZM-2.

Ladite zéolithe IZM-2 sous sa forme brute de synthèse est soumise à quatre échanges ioniques dans une solution de $NH_4NO_3$ 10N, à environ 100°C pendant 4 heures pour chaque échange de manière à obtenir ladite zéolithe IZM-2 sous forme hydrogène. Elle est ensuite mise en forme par extrusion avec un gel d'alumine. Les extrudés obtenus subissent une calcination dite sèche à 550°C sous flux d'air sec durant 10 heures de manière à éliminer le structurant organique. 1% poids de platine est déposé sur lesdits extrudés par imprégnation à sec à partir de $Pt(NH_3)_4Cl_2$. Après mise à l'étuve (110°C, 12h) et calcination sous air (21/h/g) à 420°C, on obtient le catalyseur C5 constitué de 79,10 % poids de zéolithe IZM-2, 19,90 % poids d'alumine et 1 % poids de platine. Ladite zéolithe IZM-2 présente une composition chimique de formule $SiO_2$ : 0,0067 $Al_2O_3$ (a = 0,0067) soit un rapport Si/Al égal à 75.

Exemple 11 : performances catalytiques du catalyseur C5 en hydroisomérisation des paraffines (invention)

Le catalyseur C5 est d'abord réduit sous hydrogène après chargement *in situ* dans la zone réactionnelle : après un palier d'une heure à 150°C, le catalyseur est réduit à 450°C pendant 1 heure sous débit d'hydrogène (15l/h/g).
La charge employée pour la mise en oeuvre du test d'hydroisomérisation est composée de 25% pds de nC5, 35% pds de nC6 et 40% pds de nC7. Elle est introduite dans la zone réactionnelle contenant 150 g de catalyseur C5 réduit. Le test catalytique a été réalisé dans les conditions opératoires suivantes :

- T = 230 °C ;
- P totale = 3MPa ;
- H2/HC (molaire) = 1,5 ;
- Débit H2 = 4,5 $10^{-9}$ l/h.
- pph = 1,01 $h^{-1}$ (masse de charge / masse de catalyseur / h)

Les résultats obtenus sont présentés dans le tableau 5. Ils sont exprimés en conversion des différents constituants de la charge (la conversion correspondant au pourcentage massique de chacun des constituants consommés) et en rendement C5+ exprimant la quantité massique de produits C5+ dans les effluents, le complément à 100% correspond à des produits C5- issus du craquage des constituants de la charge.

Tableau 5 : performances du catalyseur C5 en hydroisomérisation des paraffines

| | |
|---|---|
| conversion nC5 | 15% |
| conversion nC6 | 37% |
| conversion nC7 | 56,6 % |

(suite)

| | |
|---|---|
| rendement C5+ | 92,5 % |

Le catalyseur C5 selon l'invention conduit à un rendement optimal en C5+ ce qui signifie que l'effluent de sortie contient des produits présentant un bon indice d'octane. La valeur optimale du rendement en C5+ démontre que les réactions de craquage sont minimisées (seulement 7,5 % de produits non valorisables sont formés) favorisant la sélectivité envers les produits isomérisés, produits cibles de la réaction.

## Revendications

1. Catalyseur comprenant au moins une zéolithe IZM-2, au moins une matrice et au moins un métal choisi parmi les métaux des groupes VIII, VIB et VIIB, ladite zéolithe présentant un diagramme de diffraction des rayons X incluant au moins les raies inscrites dans le tableau ci-dessous :

| 2 thêta (°) | $d_{hkl}$ (Å) | Irel | 2 thêta (°) | $d_{hkl}$ (Å) | Irel |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | f |
| 14,76 | 6,00 | ff | 24,65 | 3,61 | ff |
| 15,31 | 5,78 | ff | 26,78 | 3,33 | f |
| 15,62 | 5,67 | ff | 29,33 | 3,04 | ff |
| 16,03 | 5,52 | ff | 33,06 | 2,71 | ff |
| 17,60 | 5,03 | ff | 36,82 | 2,44 | ff |
| 18,22 | 4,87 | ff | 44,54 | 2,03 | ff |

où FF = très fort ; F = fort ; m = moyen ; mf = moyen faible ; f = faible ; ff = très faible et présentant une composition chimique, exprimée sur une base anhydre, en termes de moles d'oxydes, par la formule générale suivante : $XO_2$ : $aY_2O_3$ : $bM_{2/n}O$ dans laquelle X représente au moins un élément tétravalent, Y représente au moins un élément trivalent et M est au moins un métal alcalin et/ou un métal alcalino-terreux de valence n, a et b représentant respectivement le nombre de moles de $Y_2O_3$ et $M_{2/n}O$ et a est compris entre 0,001 et 0,5 et b est compris entre 0 et 1.

2. Catalyseur selon la revendication 1 tel que X est le silicium et Y est l'aluminium.

3. Catalyseur selon la revendication 1 ou la revendication 2 tel que ladite zéolithe IZM-2 se trouve sous sa forme protonée dans laquelle la proportion en cation autre que $H^+$ est inférieure à 30% du nombre total de cations sur la zéolithe.

4. Catalyseur selon l'une des revendications 1 à 3 tel que ledit métal du groupe VIB est le chrome ou le molybdène.

5. Catalyseur selon l'une des revendications 1 à 4 tel que ledit métal du groupe VIIB est le rhénium.

6. Catalyseur selon l'une des revendications 1 à 5 tel que ledit métal du groupe VIII est choisi parmi le palladium, le nickel et le platine.

7. Catalyseur selon l'une des revendications 1 à 6 tel que ladite matrice contient de l'alumine.

8. Catalyseur selon l'une des revendications 1 à 7 tel qu'il comprend au moins un métal additionnel choisi parmi les

métaux des groupes IIIA et IVA.

**9.** Catalyseur selon l'une des revendications 1 à 8 tel qu'il se présente sous forme de billes ou d'extrudés.

**10.** Procédé d'isomérisation d'une coupe contenant au moins un composé aromatique à huit atomes de carbone par molécule, ledit procédé comprenant la mise en contact de ladite coupe aromatique avec au moins un catalyseur selon l'une des revendications 1 à 9 présent dans au moins un réacteur catalytique.

**11.** Procédé de transalkylation d'hydrocarbures alkylaromatiques pour produire des xylènes, ledit procédé comprenant la mise en contact desdits hydrocarbures alkylaromatiques avec au moins un catalyseur selon l'une des revendications 1 à 9 lequel est présent dans au moins un réacteur catalytique.

**12.** Procédé d'hydroisomérisation des paraffines présentes dans une charge comprenant en majeure partie des paraffines linéaires contenant de 5 à 8 atomes de carbone par molécule, ledit procédé comprenant la mise en contact de ladite charge avec au moins un catalyseur selon l'une des revendications 1 à 9.

**13.** Procédé de transformation d'au moins un composé aliphatique ayant de 1 à 18 atomes de carbone et portant une fonction alcool, ledit procédé étant réalisé en présence d'au moins un catalyseur selon l'une des revendications 1 à 9.

**14.** Procédé selon la revendication 13 tel que ladite transformation mise en jeu est une réaction de déshydratation au cours de laquelle ledit composé aliphatique portant une fonction alcool est déshydraté en oléfine(s) avec production d'eau.

**15.** Procédé selon la revendication 13 tel que ladite transformation mise en jeu réalise simultanément dans un même réacteur la déshydratation dudit composé aliphatique en oléfine(s) et l'oligomérisation de(s)dite(s) oléfine(s).

## Claims

**1.** A catalyst comprising at least one IZM-2 zeolite, at least one matrix and at least one metal selected from metals from groups VIII, VIB and VIIB, said zeolite having an X ray diffraction diagram including at least the peaks recorded in the table below:

| 2 theta (°) | $d_{hkl}$ (Å) | $I_{rel}$ | 2 theta (°) | $d_{hkl}$ (Å) | $I_{rel}$ |
|---|---|---|---|---|---|
| 5.07 | 17.43 | Vw | 19.01 | 4.66 | Vw |
| 7.36 | 12.01 | Vs | 19.52 | 4.54 | Vw |
| 7.67 | 11.52 | Vs | 21.29 | 4.17 | M |
| 8.78 | 10.07 | S | 22.44 | 3.96 | W |
| 10.02 | 8.82 | Vw | 23.10 | 3.85 | Mw |
| 12.13 | 7.29 | Vw | 23.57 | 3.77 | W |
| 4.76 | 6.00 | Vw | 24.65 | 3.61 | Vw |
| 15.31 | 5.78 | Vw | 26.78 | 3.33 | W |
| 15.62 | 5.67 | Vw | 29.33 | 3.04 | Vw |
| 16.03 | 5.52 | Vw | 33.06 | 2.71 | Vw |
| 17.60 | 5.03 | Vw | 36.82 | 2.44 | Vw |
| 18.22 | 4.87 | Vw | 44.54 | 2.03 | Vw |

in which: Vs = very strong; S = strong; M = medium; Mw = medium weak; W = weak; Vw = very weak, and having a chemical composition expressed as the anhydrous base in terms of moles of oxides by the following general formula: $XO_2$: $aY_2O3$: $bM_{2/n}0$, in which X represents at least one tetravalent element, Y represents at least one trivalent element and M is at least one alkali metal and/or alkaline-earth metal with valency n, a and b respectively

representing the number of moles of $Y_2O_3$ and $M_{2/n}O$; and a is in the range 0.001 to 0.5, b is in the range 0 to 1.

**2.** A catalyst according to claim 1, in which X is silicon and Y is aluminium.

**3.** A catalyst according to claim 1 or claim 2, in which said IZM-2 zeolite is in its protonated form in which the proportion of cations other than $H^+$ is less than 30% of the total number of cations on the zeolite.

**4.** A catalyst according to one of claims 1 to 3, in which said metal from group VIB is chromium or molybdenum.

**5.** A catalyst according to one of claims 1 to 4, in which said metal from group VIIB is rhenium.

**6.** A catalyst according to one of claims 1 to 5, in which said metal from group VIII is selected from palladium, nickel and platinum.

**7.** A catalyst according to one of claims 1 to 6, in which said matrix contains alumina.

**8.** A catalyst according to one of claims 1 to 7, comprising at least one additional metal selected from metals from groups IIIA and IVA.

**9.** A catalyst according to one of claims 1 to 8, in the form of beads or extrudates.

**10.** A process for the isomerization of a cut containing at least one aromatic compound containing eight carbon atoms per molecule, said process comprising bringing said aromatic cut into contact with at least one catalyst in accordance with one of claims 1 to 9 present in at least one catalytic reactor.

**11.** A process for the transalkylation of alkylaromatic hydrocarbons to produce xylenes, said process comprising bringing said alkylaromatic hydrocarbons into contact with at least one catalyst in accordance with one of claims 1 to 9 which is present in at least one catalytic reactor.

**12.** A process for the hydroisomerization of paraffins present in a feed comprising, as a major portion, linear paraffins containing 5 to 8 carbon atoms per molecule, said process comprising bringing said feed into contact with at least one catalyst in accordance with one of claims 1 to 9.

**13.** A process for the transformation of at least one aliphatic compound containing 1 to 18 carbon atoms and carrying an alcohol function, said process being carried out in the presence of at least one catalyst in accordance with one of claims 1 to 9.

**14.** A process according to claim 13, in which said transformation which is carried out is a dehydration reaction during which said aliphatic compound carrying an alcohol function is dehydrated into olefin(s) with the production of water.

**15.** A process according to claim 13, in which said transformation simultaneously carries out, in the same reactor, dehydration of said aliphatic compound into olefin(s) and oligomerization of said olefin(s).

## Patentansprüche

**1.** Katalysator, umfassend mindestens ein IZM-2-Zeolith, mindestens eine Matrix und mindestens ein Metall, das aus den Metallen der Gruppen VIII, VIB et VIIB ausgewählt ist, wobei der Zeolith ein Röntgenbeugungsdiagramm aufweist, welches mindestens die Signale umfasst, die in der untenstehenden Tabelle aufgeführt sind:

| 2 theta (°) | dhkl (Å) | Irel | 2 theta (°) | dhkl (Å) | Irel |
|---|---|---|---|---|---|
| 5,07 | 17,43 | SS | 19,01 | 4,66 | ss |
| 7,36 | 12,01 | SS | 19,52 | 4,54 | ss |
| ,67 | 11,52 | SS | 21,29 | 4,17 | m |
| 8,78 | 10,07 | S | 22,44 | 3,96 | s |

(fortgesetzt)

| 2 theta (°) | dhkl (Å) | Irel | 2 theta (°) | dhkl (Å) | Irel |
|---|---|---|---|---|---|
| 0,02 | 8,82 | SS | 23,10 | 3,85 | ms |
| 12,13 | 7,29 | SS | 23,57 | 3,77 | s |
| ,76 | 6,00 | SS | 24,65 | 3,61 | ss |
| 5,31 | 5,78 | SS | 26,78 | 3,33 | s |
| 5,62 | 5,67 | SS | 29,33 | 3,04 | ss |
| 6,03 | 5,52 | SS | 33,06 | 2,71 | ss |
| 7,60 | 5,03 | SS | 36,82 | 2,44 | ss |
| 8,22 | 4,87 | SS | 44,54 | 2,03 | ss |

wobei SS = sehr stark; S = stark; m = mittel; ms = mittelschwach; s = schwach; ss = sehr schwach ist und wobei er eine chemische Zusammensetzung aufweist, die auf einer wasserfreien Basis, in Mol an Oxiden, durch die folgende allgemeine Formel ausgedrückt wird: $XO_2 : aY_2O_3 : bM_{2/n}O$, wobei X mindestens ein vierwertiges Element darstellt, Y mindestens ein dreiwertiges Element darstellt und M mindestens ein Alkalimetall und/oder Erdalkalimetall der Wertigkeit n ist, wobei a und b die Anzahl der Mole an $Y_2O_3$ beziehungsweise an $M_{2/n}O$ darstellen und a im Bereich von 0,001 bis 0,5 liegt und b im Bereich von 0 bis 1 liegt.

**2.** Katalysator nach Anspruch 1, derart, dass X Silicium ist und Y Aluminium ist.

**3.** Katalysator nach Anspruch 1 oder Anspruch 2, derart, dass, der IZM-2-Zeolith ist seiner protonierten Form vorliegt, wobei der Anteil an Kationen, die sich von $H^+$ unterschieden, bei dem Zeolith weniger als 30 % der Gesamtanzahl an Kationen ausmacht.

**4.** Katalysator nach einem der Ansprüche 1 bis 3, derart, dass es sich bei dem Metall der Gruppe VIB um Chrom oder Molybdän handelt.

**5.** Katalysator nach einem der Ansprüche 1 bis 4, derart, dass es sich bei dem Metall der Gruppe VIIB um Rhenium handelt.

**6.** Katalysator nach einem der Ansprüche 1 bis 5, derart, dass das Metall der Gruppe VIII aus Palladium, Nickel und Platin ausgewählt ist.

**7.** Katalysator nach einem der Ansprüche 1 bis 6, derart, dass die Matrix Aluminiumoxid enthält.

**8.** Katalysator nach einem der Ansprüche 1 bis 7, derart, dass er mindestens ein zusätzliches Metall umfasst, das aus den Metallen der Gruppen IIIA und IVA ausgewählt ist.

**9.** Katalysator nach einem der Ansprüche 1 bis 8, derart, dass er in Form von Kügelchen oder Extrudatstücken vorliegt.

**10.** Verfahren zur Isomerisierung einer Fraktion, die mindestens eine aromatische Verbindung mit acht Kohlenstoffatome pro Molekül enthält, wobei die aromatische Fraktion im Rahmen des Verfahrens mit mindestens einem Katalysator nach den Ansprüchen 1 bis 9, welcher in mindestens einem Katalysereaktor vorliegt, in Kontakt gebracht wird.

**11.** Verfahren zur Transalkylierung von alkylaromatischen Kohlenwasserstoffen zwecks Herstellung von Xylolen, wobei die alkylaromatischen Kohlenwasserstoffe im Rahmen des Verfahrens mit mindestens einem Katalysator nach einem der Ansprüche 1 bis 9, welcher in mindestens einem Katalysereaktor vorliegt, in Kontakt gebracht werden.

**12.** Verfahren zur Hydroisomerisierung der Paraffine, die in einer Charge vorliegen, welche größtenteils geradkettige Paraffine mit 5 bis 8 Kohlenstoffatomen pro Molekül umfasst, wobei die Charge im Rahmen des Verfahrens mit mindestens einem Katalysator nach einem der Ansprüche 1 bis 9 in Kontakt gebracht wird.

**13.** Verfahren zur Umwandlung mindestens einer aliphatischen Verbindung, die 1 bis 18 Kohlenstoffatome und eine

funktionelle Alkoholgruppe aufweist, wobei das Verfahren in Gegenwart mindestens eines Katalysators nach einem der Ansprüche 1 bis 9 durchgeführt wird.

**14.** Verfahren nach Anspruch 13, derart, dass es sich bei der betreffenden Umwandlung um eine Dehydratisierungsreaktion handelt, im Laufe derer die aliphatische Verbindung, welche eine funktionelle Alkoholgruppe aufweist, unter Freisetzung von Wasser zu (einem) Olefin(en) dehydratisiert wird.

**15.** Verfahren nach Anspruch 13, derart, dass die betreffende Umwandlung in ein und demselben Reaktor gleichzeitig die Dehydratisierung der aliphatischen Verbindung zu (einem) Olefin(en) und die Oligomerisierung des/der Olefins/Olefine bewirkt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1953118 A